# EUROPEAN PATENT APPLICATION

(11) **EP 4 437 856 A1**
(43) Date of publication of application: **02.10.2024**
(21) Application number: 23192090.1
(22) Date of filing: 18.08.2023
(51) Int. Cl.: A23L 5/20, C12C 11/00, C12H 1/00, C12N 9/50, C12N 9/58, C12R 1/885, C07K 5/04, C12C 5/00, C12C 3/00, C12G 3/08, C12C 7/28

(54) **NOVEL ENZYME COMPOSITION AND NOVEL BEER BREWING PROCESS**

(30) Priority: 31.03.2023 EP 23165916; 11.04.2023 EP 23167456; 24.07.2023 EP 23187177
(71) Applicant: DSM IP Assets B.V., 6221 BE Maastricht (NL)
(72) Inventor: DEKKER, Petrus Jacobus Theodorus, 6100 AA Echt (NL); SMAL, Pierre-Lambert, 6100 AA Echt (NL); DE JONG, René Marcel, 6100 AA Echt (NL); TABELING, Michael Dennis, 6100 AA Echt (NL)
(74) Representative: dsm-firmenich IP

(57) **Abstract**

A composition, for addition to a process for the production of an alcoholic beverage for human consumption, comprising or consisting of:
- a first enzymatic component, comprising or consisting of a glutamine-specific protease and/or prolyl-specific protease, preferably a glutamine-specific endoprotease and/or prolyl-specific endoprotease, most preferably a prolyl-specific endoprotease; and
- a second enzymatic component, comprising or consisting of a cellulolytic enzyme, preferably cellulase,
wherein the second enzymatic component is derived from and/or produced by a *Trichoderma reesei.*

## Description

### Field of the invention

The invention relates to a novel enzyme composition and novel beer brewing process.

### Background of the invention

Traditional beer production processes comprise a first hydrolysis stage in which starch is hydrolysed into saccharides and a second separate fermentation stage where these saccharides are subsequently fermented into alcohol. The fermentation is traditionally carried out with yeast cells.

For example, WO2013/167573 describes a method of preparing a wort with a high level of free amino acids, said method comprising the steps of a) mashing a composition comprising barley in the presence of exogenous enzymes comprising an alpha amylase, a beta glucanase, a pullulanase, a xylanase, and a lipase; and b) adding to said composition during mashing or after completion of mashing at least two different exogenous proteases, wherein one protease has endoprotease activity, and the other protease has exopeptidase activity. The endoprotease could be a metalloprotease, a proline-endoprotease or a glutamine endoprotease. In example 2, Ondea Pro (commercially available from Novozymes), a metalloprotease and an exopeptidase were added in a mashing step. According to WO2013/167573 mashing is the process of converting starch from the milled barley malt and solid adjuncts into fermentable and unfermentable sugars to produce wort of the desired composition. After mashing when all the starch has been broken down, it was necessary to separate the liquid extract (the wort) from the residual solids (spent grains) and boil it. Hereby numerous substances including several proteins were denatured. After cooling and removal of precipitates, the wort was aerated and fermented to produce a beer. Due to the boiling, no alive enzymes could be present during the fermentation.

However, more recently home brewers have also started to use a so-called "SSF" (simultaneous saccharification and fermentation) process. In this process the hydrolysis step (also referred to as saccharification step) and the fermentation step are carried out simultaneously.

An example of such a simultaneous saccharification and fermentation process is described by Baltaci et al., in their article titled " The simultaneous saccharification and fermentation of malt dust and use in the acidification of mash", published online DOI 10.1002/jib.554 on 26 February 2019. They describe how an SSF process combines the saccharification of cellulosic materials using cellulolytic enzymes, which generate the sugars, and fermentation which consumes them. The process is also preferred as it reduces reaction volume and process time. Baltaci et al. discuss the issues of malt dust and the acidification of the mash in the process.

In addition, some special alcoholic beverages such as sake may be produced in a process where saccharification and fermentation occur simultaneously within the same brewing tank.

Haze is a well-known phenomenon in the beer industry. In a beer brewing process haze formation can occur at different stages. In "Enzymes in food processing" edited by T. Nagodawithana and G. Reed, 3rd edition, Academic press Inc., San Diego, Chapter V, p.448-449, it has been proposed that the haze in beer is the result of interactions between beer proteins and polyphenolic procyanidins.

WO2002046381 describes a method for the prevention or reduction of haze in a beverage wherein a prolyl-specific endoprotease (also referred to as "PEP") is added to the beverage. WO2002046381 explains that the activity of prolyl-specific endoproteases is dependent on the pH. It describes for example that an endoprotease is added to a beverage having a maximum prolyl specific activity at a pH which corresponds to the pH of the beverage it is added to. Since haze formation often occurs in acidic beverages such as for example beer, wine and fruit juice, prolyl-specific endoproteases having a prolyl specific activity at a pH value below 7 are preferably used. WO2002046381 subsequently provides several specific prolyl- specific endoproteases that have since then been very successful in reduction of haze in beverages.

The process as described in WO2002046381 provides a good solution for the haze in traditional beer brewing processes.

However, in the above described SSF processes, the "haze" in the beer may have a different composition and may be caused by a mixture of components. The haze may not only comprise the above mentioned interactions between beer proteins and polyphenolic procyanidins, but may also comprise fine particulate matter and/or polysaccharides resulting from the feedstock. The role of polysaccharides in haze formation is not well understood, but studies have shown effects of the polysaccharides on this haze formation.

The provision of an enzyme composition and/or a process allowing one to speed up or otherwise improve a SSF process; and/or to speed-up or otherwise improve the removal of fine particulate matter in the fermentation broth and/or fermentation tank; and/or to improve the taste of the alcoholic beverage product would be an advancement in the art.

### Summary of the invention

A novel enzyme composition, a novel process applying such novel enzyme and a novel method for producing such novel enzyme composition allowing one to speed up or otherwise improve a SSF process and/or to speed-up or otherwise improve the removal of fine particulate matter in the fermentation broth and/or fermentation tank and/or to improving the taste of the alcoholic beverage product, has now been found.

Accordingly, in a first aspect the invention provides a composition (herein also referred to as "enzyme composition"), for addition to a process for the production of an alcoholic beverage for human consumption, comprising or consisting of:
- a first enzymatic component, comprising or consisting of a glutamine-specific protease and/or prolyl-specific protease, preferably a glutamine-specific endoprotease and/or prolyl-specific endoprotease, most preferably a prolyl-specific endoprotease; and
- a second enzymatic component, comprising or consisting of a cellulolytic enzyme, preferably cellulase,
wherein the second enzymatic component is derived from and/or produced by a *Trichoderma reesei.*

Preferably, the cellulolytic enzyme, preferably cellulase, is derived and/or produced from *Trichoderma reesei.* As illustrated by the examples, a cellulolytic enzyme, suitably cellulase, that is derived and/or produced from *Trichoderma reesei* produces the best results. More preferably the first enzymatic component and the second enzymatic component are both derived and/or produced from the same organism, preferably, *Trichoderma reesei,* which provides the benefit of an efficient production.

In a second aspect the invention provides a process for the preparation comprising the use of the above novel enzyme composition.

In a third aspect the invention provides a process for the production of the above novel enzyme composition.

Without wishing to be bound by any kind of theory, it is believed that the cellulase can be helpful to convert small residues of the feedstock in a SSF process and that the above invention can therefore help to improve to speed up or otherwise improve a home-brewing SSF process and/or speed-up or otherwise improve the removal of fine particulate matter in the fermentation broth and/or fermentation tank and/or improving the taste of a (home-made) beer would be an advancement in the art.

The invention is illustrated by the examples.

The above novel enzyme composition can advantageously be useful in a process for the production of an alcoholic beverage for human consumption, preferably a beer. Therefore there is advantageously provided a novel process for the production of an alcoholic beverage for human consumption, preferably a novel beer brewing process, using the above novel enzyme composition.

In a fourth aspect, the present invention thus provides a process for the production of an alcoholic beverage for human consumption, preferably a beer brewing process, comprising the addition of a composition as described above or the addition of a bacterial or fungal strain, preferably a *Trichoderma reesei* strain, as described below.

More preferably the present invention provides a process for the production of an alcoholic beverage for human consumption, preferably a beer brewing process, comprising the addition of a composition as described above, wherein the composition comprises or consists of:
- a first enzymatic component, comprising or consisting of glutamine-specific protease and/or prolyl-specific protease, preferably a glutamine-specific endoprotease and/or prolyl-specific endoprotease, most preferably a prolyl-specific endoprotease; and
- a second enzymatic component, comprising or consisting of a cellulolytic enzyme, preferably cellulase.

Further, in a fifth aspect, the present invention provides an alcoholic beverage for human consumption obtained or obtainable by the above process.

### Drawings

The invention is illustrated with the following figures.

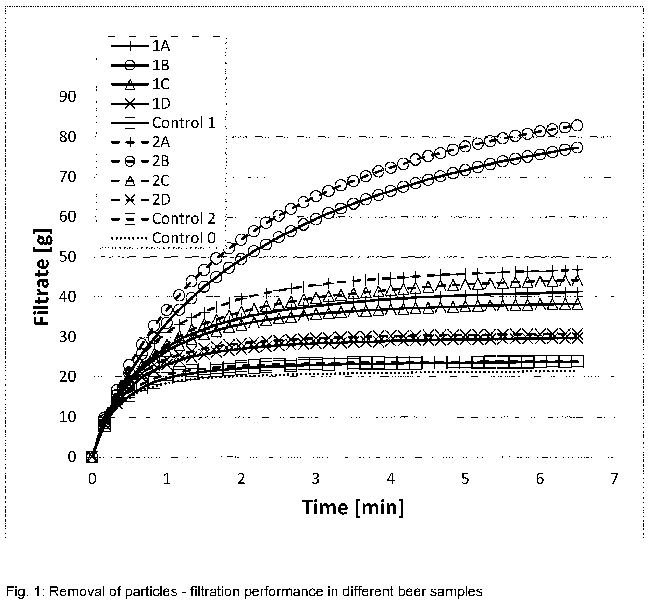

Figure 1, illustrating the removal of particles - filtration performance in different beer samples.

### Detailed description of the invention

### Definitions

Unless defined otherwise or clearly indicated by context, all technical and scientific terms used herein have the same meaning as commonly understood by one of ordinary skill in the art.

Throughout the present specification and the accompanying claims, the words "comprise" and "include" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element. When referring to a noun (for example a compound, an additive, etc.) in the singular, the plural is meant to be included. Thus, when referring to a specific moiety, for example a "strain", this means "at least one" of that strain, for example "at least one strain", unless specified otherwise.

When referring to a compound of which several isomers exist (for example, a D and an L enantiomer), the compound in principle includes all enantiomers, diastereomers and cis/trans isomers of that compound that may be used in the particular aspect of the invention; in particular when referring to such as compound, it includes the natural isomer(s).

Unless explicitly indicated otherwise, the various embodiments of the invention described herein can be cross-combined.

The terms "peptide" and "oligopeptide" are considered synonymous (as is commonly recognized) and each term can be used interchangeably as the context requires to indicate a chain of at least two amino acids coupled by peptidyl linkages. The word "polypeptide" is used herein for chains containing more than seven amino acid residues. All oligopeptide and polypeptide formulas or sequences herein are written from left to right and in the direction from amino terminus to carboxy terminus. References to amino acid names herein refer to the amino acids as described in the handbook by Jeremy M. Berg, John L. Tymoczko and Lubert Stryer, titled Biochemistry, 6th edition, published 2007 by W.H. Freeman and Company, New York, USA, Chapter 2. The one-letter code of amino acids used herein is commonly known in the art and can be found for example in the handbook by Jeremy M. Berg, John L. Tymoczko and Lubert Stryer, titled Biochemistry, 6th edition, published 2007 by W.H. Freeman and Company, New York, USA, Chapter 2, Table 2.2.

Nucleic acid sequences (i.e. polynucleotides) or proteins (i.e. polypeptides) may be native or heterologous to the genome of the host cell.

"Native", "homologous" or "endogenous" with respect to a host cell, means that the nucleic acid sequence does naturally occur in the genome of the host cell or that the protein is naturally produced by that cell. The terms "native", "homologous" and "endogenous" are used interchangeable herein.

As used herein, "heterologous", with respect to the host cell, may refer to a polynucleotide that does not naturally occur in that way in the genome of the host cell or that a polypeptide or protein is not naturally produced in that manner by that cell. Heterologous protein expression involves expression of a protein that is not naturally expressed in that way in the host cell. The term "heterologous expression" refers to the expression of heterologous nucleic acids in a host cell.

As used herein "promoter" is a DNA sequence that directs the transcription of a (structural) gene or other (part of) nucleic acid sequence. Suitably, a promoter is located in the 5'-region of a gene, proximal to the transcriptional start site of a (structural) gene. Promoter sequences may be constitutive, inducible or repressible. In an embodiment there is no (external) inducer needed.

### The first enzyme component

The composition according to the invention suitably comprises a first enzymatic component, comprising or consisting of a glutamine-specific protease and/or prolyl-specific protease, preferably a glutamine-specific endoprotease and/or prolyl-specific endoprotease, most preferably a prolyl-specific endoprotease.

The wording "protease", "protease enzyme", "enzyme having protease activity", "protein having protease activity" and "polypeptide having protease activity", "peptidase", "peptidase enzyme", "enzyme having peptidase activity", "protein having peptidase activity" and "polypeptide having peptidase activity" are used interchangeably herein.

Also the wording "endoprotease", "endoprotease enzyme", "enzyme having endoprotease activity", "protein having endoprotease activity" and "polypeptide having endoprotease activity", "endopeptidase", "endopeptidase enzyme", "enzyme having endopeptidase activity", "protein having endopeptidase activity" and "polypeptide having endopeptidase activity" are used interchangeably herein.

Further the wording "prolyl-specific" and "proline-specific" are used interchangeably herein. Most preferably the first enzymatic component comprises or consists of a prolyl-specific endoprotease.

The glutamine-specific protease and/or proline-specific protease, respectively glutamine-specific endoprotease and/or proline-specific endoprotease, may be used in the invention in an isolated or purified form. By "isolated" or "purified" is intended a proline-specific protease removed from its native environment. For example, recombinantly produced proline-specific protease expressed in host cells are considered isolated for the purpose of the invention as are native or recombinant polypeptides which have been substantially purified by any suitable technique such as, for example, the single-step purification method disclosed in Smith and Johnson, Gene 67:31-40 (1988).

A proline-specific protease suitable for use in the invention may be recovered from recombinant cell cultures by methods well-known to those skilled in the art, including for example ammonium sulfate or ethanol precipitation, acid extraction and chromatographic methods such as high performance liquid chromatography (HPLC).

A proline-specific protease suitable for use in the invention may be a naturally-purified product, a product or a chemical synthesis, a product produced by a recombinant technique from a prokaryotic or eukaryotic host, including, for example. Bacterial, yeast, fungal, higher plant, insect and mammalian cells.

Preferably the first enzymatic component is derived from and/or produced by a micro-organism, preferably a bacterium or fungus, most preferably a Trichoderma reesei. That is, preferably the proline-specific protease, respectively the proline specific endoprotease, derived from and/or produced by a micro-organism, preferably a fungus, most preferably a Trichoderma reesei.

It will be understood though that the protease may be mixed with carriers or diluents which will not interfere with the intended purpose of the enzyme and still be regarded as isolated. A proline-specific protease suitable for use in the invention may also be in a more substantially purified form. Accordingly, the proline-specific protease may be comprised in a preparation in which more than 70%, for example more than 80%, 90%, 95%, 98% or 99% of any proteases present in the preparation is a proline-specific protease. Most preferably, the proline-specific protease may be in a form free from or substantially free from any other protease.

A proline-specific protease suitable for use in the invention may be used in an immobilized form so that large quantities of protein containing liquids can be treated. Ways to select appropriate support materials and suitable immobilization methods have been extensively described in the literature, for example in "Immobilization of Enzymes and Cells" (ed. Gordon F. Bickerstaff; ISBN 0-89603-386-4).

In the context of the present invention, a "prolyl-specific", respectively "proline-specific", protease or endoprotease is defined as a protease, respectively endoprotease, that cuts proteins or peptides at places where the protein or peptide contains a proline residue in its chain. The wording prolyl-specific" and "proline-specific" are used interchangeably herein. Preferably, the "prolyl-specific", respectively "proline-specific" protease, is an endoprotease that "cuts" (hydrolyses) proteins or peptides at places where the protein or peptide contains a proline residue. In the method according to the invention, a proline-specific endoprotease is preferably used that hydrolyses the peptide bond at the carboxyterminal end of proline residues. Examples of such enzymes are prolyl oligopeptidases (EC 3.4.21.26) as well as the Aspergillus niger derived prolyl endoprotease reported in J. Agic Food Chem, Vol 53 (20), 7950-7957, 2005) and proline-specific dipeptidyl peptidases such as DPP IV (EC 3.4.14.5). A proline-specific endoprotease that cuts proline-residues at their NH₂-terminus is for example described in a publication in Nature of 15 January 1998, Vol.391, p.301-304.

In the context of the present invention, a "glutamine-specific" protease is a protease that cuts proteins or peptides at places where the protein or peptide contains a glutamine residue in its chain. Preferably, the glutamine-specific protease, is a glutamine-specific endoprotease that "cuts" (hydrolyses) proteins or peptides at places where the protein or peptide contains a glutamine residue.

In an especially preferred embodiment, the first enzymatic component comprises or consists of a protease, preferably an endoprotease, which is both glutamine-specific as well as proline-specific.

In a further aspect the invention thus also provides a composition comprising or consisting of:
- a first enzymatic component, comprising or consisting of a protease, preferably an endoprotease, and
- a second enzymatic component, comprising or consisting of a cellulolytic enzyme, preferably cellulase,
wherein preferably the protease, respectively the endoprotease, is:
- capable of hydrolyzing a substrate protein or peptide at one or more glutamine residues; and/or
- capable of hydrolyzing a substrate protein or peptide at one or more proline residues.
Preferences for such a composition and the first and second enzymatic component are further as described above and below.

Examples of such an enzyme with both glutamine-specific action as well as proline-specific action are the enzymes as described in WO2022/266456. The protease, respectively endoprotease, of the first enzymatic component may also be a "precursor" as described hydrolyzing a substrate protein or peptide at one or more glutamine residues.

In a first preferred embodiment the first enzymatic component includes one or more enzymes having an amino acid sequence that has at least 75, 80, 85, 90, 95, 98, 99 or 100% sequence identity to one or more of SEQ ID NO:1, SEQ ID NO:2, SEQ ID NO:3, SEQ ID NO:4 and/or SEQ ID NO: 16 as described in WO2022/266456 or an endoprotease active fragment thereof such as a mature protein. The sequence listing of these enzymes as provided with WO2022/266456 is incorporated herewith by reference. These enzymes may be produced as described in WO2022/266456.

In a second preferred embodiment the first enzymatic component includes one or more enzymes having an amino acid sequence that has at least 75, 80, 85, 90, 95, 98, 99 or 100% sequence identity to one or more of SEQ ID NO:4, SEQ ID NO:6, SEQ ID NO:8 as described in WO2020/025704 or an endoprotease active fragment thereof such as a mature protein. The sequence listing of these enzymes as provided with WO2020/025704 is incorporated herewith by reference. These enzymes may be produced as described in WO2020/025704.

Preferred examples further include the proline-specific proteases described in:
- the article titled "Crystal structure and substrate recognition mechanism of the prolyl endoprotease PEP from Aspergillus niger" by Miyazono et al, published in Biochemical and Biophysical Research Communications, Volume 591, 5 February 2022, Pages 76-81; and
- in the article titled "Extracellular Prolyl Endoprotease from Aspergillus niger and Its Use in the Debittering of Protein Hydrolysates" by Edens et al, published in J. Agric. Food Chem. 2005, 53, 7950-7957; and
- In the article titled " Influence of dietary components on Aspergillus niger prolyl endoprotease mediated gluten degradation" by Montserrat et al., published in Biochemical and Biophysical Research Communications 591 (2022) pages 76-81,
each incorporated herein by reference.

Preferably the composition comprises a first enzymatic component, wherein this first enzymatic component preferably has its optimum activity at a pH in the range from equal to or more than pH 1.0, more preferably from equal to or more than pH 1.2 to equal to or less than pH 6.0, more preferably to equal to or less than pH 5.5, still more preferably to equal to or less than pH 5.0, even more preferably to equal to or less than pH 4.5 and most preferably to equal to or less than pH 4.0.

Hence, preferably the activity of the endoprotease, preferably glutamine-specific and/or proline-specific endoprotease, is dependent on the pH. Preferably the endoprotease, glutamine-specific and/or proline-specific endoprotease, is an endoprotease, respectively glutamine-specific and/or proline-specific endoprotease, with an acidic pH optimum, i.e. a pH optimum below pH 7.0. More preferably the endoprotease, preferably glutamine-specific and/or proline-specific endoprotease, is an endoprotease, respectively proline-specific endoprotease, having its optimum activity at a pH in the range from equal to or more than pH 1.0, more preferably from equal to or more than pH 1.2 to equal to or less than pH 6.0, more preferably to equal to or less than pH 5.0, still more preferably to equal to or lower than pH 4.0, even more preferably to equal to or lower than pH 3.0 and most preferably to equal to or less than pH 2.5. Preferably the endoprotease, preferably proline-specific endoprotease, is an endoprotease, respectively proline-specific endoprotease, having its optimum activity at a pH in the range from pH 1.0 to pH 5.0, even more preferably in the range from pH 1.2 to pH 4.5.

Most preferably the endoprotease, preferably glutamine-specific and/or proline-specific endoprotease, is an endoprotease, more preferably a prolyl-specific endoprotease, most preferably as described in EP-A-1326957, WO2005027953, WO2002046381 or WO 2022/266456, which are herein incorporated by reference.

Preferably the endoprotease, preferably glutamine-specific and/or proline-specific endoprotease, most preferably a prolyl-specific endoprotease, is selected from the group consisting of:
(a) a polypeptide which has an amino acid sequence which has equal to or more than 40%, more preferably equal to or more than 60%, still more preferably equal to or more than 70%, even more preferably equal to or more than 80%, still even more preferably equal to or more than 90%, yet even more preferably equal to or more than 95%, and most preferably equal to or more than 99% overall amino acid sequence identity with SEQ ID NO: 1, SEQ ID NO: 3, OR SEQ ID NO: 5 or a fragment thereof, each as described in WO2002046381;
(b) a polypeptide which is encoded by a polynucleotide which hybridizes with
   (i) the nucleic acid sequence of SEQ ID NO: 2 , SEQ ID NO: 4, SEQ ID NO: 6 or a fragment thereof which is at least 80% or 90% identical over 60, preferably over 100 nucleotides, more preferably at least 90% identical over 200 nucleotides, each as described in WO2002046381 ,or
   (ii) a nucleic acid sequence complementary to the nucleic acid sequence of (i).

The so-called active site(s) within an enzyme are those parts of the enzyme that are responsible for interaction with the substrate. This interaction may for example comprise the binding of and/or catalysing of the reaction in the substrate. The formation and presence of such so-called active site(s) can be dependent on the enzyme's primary, secondary and/or tertiary structure. Examples of active sides include catalytic triad(s) and/or an oxyanion hole(s).

Preferably the endoprotease, preferably glutamine-specific and/or proline-specific endoprotease, is an endoprotease, respectively glutamine-specific and/or proline-specific endoprotease, most preferably a prolyl-specific endoprotease, comprising a catalytic triad and/or an oxyanion hole. More preferably the endoprotease, preferably glutamine-specific and/or proline-specific endoprotease, is an endoprotease, respectively glutamine-specific and/or proline-specific endoprotease, comprising a catalytic triad and an oxyanion hole.

Preferably the catalytic triad comprises an amino acid sequence comprising or consisting of Serine, Histidine and Aspartic acid. Such a catalytic triad is herein also referred to as a Ser/Asp/His catalytic triad. In one preferred embodiment of the invention the ND1 or Nd1 nitrogen atom of side chain of the histidine amino acid in the above catalytic triad is protonated, and the NE2 or Ne2 nitrogen atom (pKₐ of 6.0) of the side chain of the histidine amino acid is unprotonated. In another preferred embodiment of the invention the histidine amino acid in the above catalytic triad is deprotonated. By deprotonated histidine is herein preferably understood a histidine amino acid that has lost its proton (H⁺ ion) on its side chain NE2 or Ne2 nitrogen atom.

By an oxyanion hole can be understood a structural feature found in the active site of certain hydrolytic enzymes that stabilizes negatively charged transition states during catalysis, for example in esterase, lipase and peptidase enzymes. Suitably an oxyanion hole can stabilize a transition state negative charge on a deprotonated oxygen or alkoxide. Stabilising this transition state may advantageously lower the activation energy necessary for the reaction and can thus promote catalysis. The oxyanion holes in hydrolytic enzymes are typically made up from hydrogen bond-donating groups of polar or basic amino acid side chains like Tyr, Trp, Arg, Lys, Asn, Gln, Ser, Thr or also often two backbone amides that hydrogen bond with the oxygen atoms of the negatively charged transition state.

However, in the composition according to the invention, the enzyme component preferably comprises or consists of an endoprotease, preferably a prolyl-specific endoprotease, comprising an oxyanion hole comprising a protonated side chain of an acidic amino acid. More preferably this oxyanion hole comprises a glutamic acid amino acid, preferably in its protonated state (Glu-H).

The proline-specific protease is preferably produced in or derived from a micro-organism, preferably a bacterium or a fungus. The proline-specific protease may be a natural occurring, recombinant or chemically modified proline-specific protease enzyme. Proline-specific proteases have for example been identified in species of *Aspergillus* (EP 0 522 428), *Flavobacterium* (EP 0 967 285) and *Aeromonas* (J.Biochem.113, 790-796), *Xanthomonas* and *Bacteroides.* The proline-specific protease of the invention may be isolated from one of the above-mentioned microbial species, particularly from a species of *Aspergillus* or *Trichoderma.* Preferably, the proline-specific endoprotease is isolated from a strain of *Aspergillus niger* or *Trichoderma reesei.* More preferably, the proline-specific endoprotease is isolated from an *Aspergillus niger* or *Trichoderma reesei* host engineered to overexpress a gene encoding a proline-specific endoprotease.

Other hosts, such as *E. coli* can also be useful. For example, the cloning and overproduction of the *Flavobacterium* derived proline-specific endoprotease in, amongst others, *E.coli* has made certain proline-specific endoproteases available in a pure form. An example of such an overproducing construct is provided in the World Journal of Microbiology & Biotechnology, Vol 11, pp 209-212.

The enzymes can be native or heterologous to the host cell. In a preferred embodiment, the enzyme can be native to an Aspergillus species and be produced in such Aspergillus species, preferably Aspergillus niger. In another preferred embodiment the enzyme can be derived from an Aspergillus species and the enzyme may be heterologously produced in another host cell, such as a Trichoderma reesei.

Preferred glutamine-specific and/or prolyl-specific endoproteases include glutamine-specific and/or prolyl-specific endoproteases derived from Aspergillus niger, Aspergillus transmontanensis, Aspergillus homomorphus, Aspergillus pseudocaelatus, Aspergillus neoauricomus, Aspergillus albertensis, Aspergillus albertensis, Aspergillus wentii, Aspergillus brasiliensis, Aspergillus sclerotioniger , Aspergillus bertholletius, Aspergillus awamori, Aspergillus tubegensis, Aspergillus accretis, Aspergillus foretidus, Aspergillus nidulans, Aspergillus japonica. Others include Oryzae and Aspergillus ficuum, and Trichoderma reesei, Fusarium graminearum, Penicillium chrysogenum, Acremonium alabrames , Myserio Photora Thermophil (Thermophilum).

Preferably the compositions as described herein are compositions, wherein the first enzymatic component is derived from and/or produced by a micro-organism, preferably a bacterium or fungus, most preferably a Trichoderma reesei.

Especially preferred herein are glutamine-specific and/or prolyl-specific endoproteases that are derived from an Aspergillus species but that are heterologously produced in a Trichoderma species, preferably Trichoderma reesei.

In another preferred embodiment, the *Aspergillus niger* host or *Aspergillus niger* or *Trichoderma reesei* host is preferably used to produce a non-recombinant self-construct utilizing *A. niger* promoters to drive the expression of a gene encoding an *A. niger* proline-specific endoprotease. That is, preferably the endoprotease, preferably proline-specific endoprotease, is a non-recombinant endoprotease, respectively non-recombinant proline-specific endoprotease.

### The second enzyme component

The composition further comprises a second enzymatic component, comprising or consisting of a cellulolytic enzyme, preferably cellulase.

The second enzymatic component is preferably derived from or produced by a micro-organism, preferably a bacterium or fungus, most preferably a Trichoderma reesei.

As used herein, a cellulolytic enzyme is an enzyme capable of partially or completely degrading cellulose. This enzymatic process is also referred to as cellulolysis. Cellulose is a polysaccharide comprising a chain of beta-1,4-linked D-glucose monomer units. Suitably a cellulose polysaccharide may comprise in the range from equal to or more than 20, more preferably equal to or more than 50, still more preferably equal to or more than 100, even more preferably equal to or more than 1000 to suitably equal to or less than 1000000, preferably equal to or less than 100000 monomer units. The beta-1,4-linkages in cellulose are challenging to break but can be broken by cellulolytic enzymes. A preferred cellulolytic enzyme is cellulase.

As used herein, a cellulase is any polypeptide having cellulase activity. A cellulase can suitably be any enzyme which is capable of hydrolysing beta-1,4-D-glucosidic linkages in cellulose . Such hydrolysis advantageously allows for the partial or complete degradation of cellulose. A polypeptide which is capable of degrading cellulose is one which is capable of catalyzing the process of breaking down cellulose into smaller units, either partially, for example into cellodextrins, or completely into glucose monomers. A cellulase according to the invention may give rise to a mixed population of cellodextrins and glucose monomers. Such degradation will typically take place by way of a hydrolysis reaction.

The terms "polypeptide having cellulase activity" "cellulase enzyme" or simply "cellulase" are used interchangeably herein. As indicated above advantageously such cellulase can hydrolyze beta-1 ,4-D-glucosidic linkages in cellulose, thereby partially or completely degrading cellulose. The cellulase can be an "exocellulase", "endocellulase" or "cellobiase". Preferably the cellulase is an "endocellulase" or "cellobiase", most preferably the cellulase is an "endocellulase".

The composition may comprise one, two, three, four, five, six or more (types of) cellulases. The composition may comprise any cellulase, for example, a lytic polysaccharide monooxygenase (e.g. GH61), a cellobiohydrolase, an endo-β-1,4-glucanase, a beta-glucosidase or a β-(1,3)(1,4)-glucanase.

As used herein, a cellobiohydrolase (EC 3.2.1.91) is any polypeptide which is capable of catalyzing the hydrolysis of 1,4-β-D-glucosidic linkages in cellulose or cellotetraose, releasing cellobiose from the ends of the chains. This enzyme may also be referred to as cellulase 1,4-β-cellobiosidase, 1,4-β-cellobiohydrolase, 1,4-β-D-glucan cellobiohydrolase, avicelase, exo-1,4-β-D-glucanase, exocellobiohydrolase or exoglucanase. Preferably the cellulase is a mixture of cellulase enzymes comprising one or more glucanase enzymes, more preferably one or more beta- glucanase enzymes.

As used herein, an endo-β-1,4-glucanase (EC 3.2.1.4) is preferred and is any polypeptide which is capable of catalyzing the endohydrolysis of 1,4-β-D-glucosidic linkages in cellulose, lichenin or cereal β-D-glucans. Such a polypeptide may also be capable of hydrolyzing 1,4-linkages in β-D-glucans also containing 1,3-linkages. This enzyme may also be referred to as cellulase, avicelase, β-1,4-endoglucan hydrolase, β-1,4-glucanase, carboxymethyl cellulase, celludextrinase, endo-1,4-β-D-glucanase, endo-1,4-β-D-glucanohydrolase, endo-1,4-β-glucanase or endoglucanase.

As used herein, a beta-glucosidase (EC 3.2.1.21) is also preferred and is any polypeptide which is capable of catalysing the hydrolysis of terminal, non-reducing β-D-glucose residues with release of β-D-glucose. Such a polypeptide may have a wide specificity for β-D-glucosides and may also hydrolyze one or more of the following: a β-D-galactoside, an α-L-arabinoside, a β-D-xyloside or a β-D-fucoside. This enzyme may also be referred to as amygdalase, β-D-glucoside glucohydrolase, cellobiase or gentobiase.

As used herein, a β-(1,3)(1,4)-glucanase (EC 3.2.1.73) is also preferred and is any polypeptide which is capable of catalysing the hydrolysis of 1,4-β-D-glucosidic linkages in β-D-glucans containing 1,3- and 1,4-bonds. Such a polypeptide may act on lichenin and cereal β-D-glucans, but not on β-D-glucans containing only 1,3- or 1,4-bonds. This enzyme may also be referred to as licheninase, 1,3-1,4-β-D-glucan 4-glucanohydrolase, β-glucanase, endo-β-1,3-1,4 glucanase, lichenase or mixed linkage β-glucanase. An alternative for this type of enzyme is EC 3.2.1.6, which is described as endo-1,3(4)-beta-glucanase. This type of enzyme hydrolyses 1,3- or 1,4-linkages in beta-D-glucanse when the glucose residue whose reducing group is involved in the linkage to be hydrolysed is itself substituted at C-3. Alternative names include endo-1,3-beta-glucanase, laminarinase, 1,3-(1,3;1,4)-beta-D-glucan 3 (4) glucanohydrolase. Substrates include laminarin, lichenin and cereal beta-D-glucans. The β-(1,3)(1,4)-glucanase (EC 3.2.1.73) respectively endo-1,3(4)-beta-glucanase [EC 3.2.1.6], are preferred as second enzymatic components.

In view of the above, the second enzymatic component preferably comprises or consists of a glucanase, more preferably a beta-glucanase (also referred to as "β-glucanase"), most preferably a beta-glucanase derived from *Trichoderma reesei.*

Suitable cellulases can be derived from a plant, an animal, or a microorganism. Preferably the cellulase is derived from a microorganism, such as a fungus or a bacterium. Preferred cellulases are those derived from a bacterium or a fungus. Most preferred is a *Trichoderma reesei* derived cellulase.

That is, preferably the second enzymatic component, preferably cellulase, more preferably a beta-glucanase, is derived from and/or produced by a micro-organism, preferably a fungus, most preferably a Trichoderma reesei.

Preferably the activity of the cellulase is dependent on the pH.

Preferably the composition comprises a second enzymatic component, wherein this second enzymatic component preferably has its optimum activity at a pH in the range from equal to or more than pH 1.0, more preferably from equal to or more than pH 1.2 to equal to or less than pH 6.0, more preferably to equal to or less than pH 5.5, still more preferably to equal to or less than pH 5.0, even more preferably to equal to or less than pH 4.5 and most preferably to equal to or less than pH 4.0.

Hence, preferably the cellulase is a cellulase with an acidic pH optimum, i.e. a pH optimum below pH 7.0. More preferably the cellulase is a cellulase having its optimum activity at a pH in the range from equal to or more than pH 1.0, more preferably from equal to or more than pH 1.2 to equal to or less than pH 6.0, more preferably to equal to or less than pH 5.0, still more preferably to equal to or lower than pH 4.0, even more preferably to equal to or lower than pH 3.0 and most preferably to equal to or less than pH 2.5. Preferably the cellulase is a cellulase having its optimum activity at a pH in the range from pH 1.0 to pH 5.0, even more preferably in the range from pH 1.2 to pH 4.5.

The cellulosic enzyme, preferably cellulase, is preferably produced in or derived from a micro-organism, preferably a bacterium or a fungus. The cellulosic enzyme, preferably cellulase, may be a natural occurring, recombinant or chemically modified cellulosic enzyme, respectively cellulase. Examples of suitable cellulases include cellulases produced in or derived from the genera *Bacillus, Pseudomonas, Streptomyces, Trichoderma, Humicola, Fusarium, Thielavia* and *Acremonium,* and also cellulases produced in or derived from *Humicola insolens, Myceliophthora thermophila* or *Fusarium oxysporum.* Preferred are cellulases produced in or derived from *Trichoderma.*

The cellulolytic enzyme, respectively cellulase, may be produced recombinantly in a heterologous expression system, such as a microbial or fungal heterologous expression system. Examples of suitable heterologous expression systems include bacterial (e.g., *E. coli, Bacillus* sp.) and eukaryotic systems. Eukaryotic systems can employ yeast (e.g., *Pichia* sp., *Saccharomyces* sp.) or fungal (e.g., *Trichoderma* sp. such as *T. reesei, Aspergillus* species such as *A. niger*) expression systems. Of these fungal expression systems, especially *Trichoderma reesei,* are preferred. For example, a cellulase may conveniently be produced such as described in U.S. Pat. Nos. 4435307, 5776757 and 7604974, herewith incorporated by reference.

Suitable examples of cellulases are described in U.S. Pat. Nos. 4435307, 5648263, 5691178, 5776757, 6562612 and 7604974 and are herewith incorporated by reference.

Preferred cellulases are the cellulases disclosed in U.S. Pat. Nos. 4689297, 5814501, 5324649, and International Patent Appl. Publ. Nos. WO92/06221 and WO92/06165, which are also herewith incorporated by reference.

Exemplary commercial cellulases that could be used in this invention include CELLUZYME^{®} and CAREZYME^{®} (Novozymes A/S); CLAZINASE^{®} and PURADAX^{®} HA (DuPont Industrial Biosciences), LAMINEX^{®} (IFF Bioscience) and KAC-500(B)^{®}(Kao Corporation).

### The enzyme composition

Preferably the composition according to the invention is a composition comprising or consisting of:
- a first enzymatic component, comprising or consisting of a prolyl-specific protease, preferably a prolyl-specific endoprotease; and
- a second enzymatic component, comprising or consisting of a cellulolytic enzyme, preferably cellulase,
wherein the molar ratio of moles cellulolytic enzyme, preferably cellulase, to moles prolyl-specific protease, preferably prolyl-specific endoprotease, is equal to or less than 1:1, more preferably equal to or less than 0.5:1, even more preferably equal to or less than 0.1:1, still more preferably equal to or less than 0.05:1 and most preferably equal to or less than 0.01:1.

More preferably the composition according to the invention is a composition comprising or consisting of:
- a first enzymatic component, comprising or consisting of a prolyl-specific protease, preferably a prolyl-specific endoprotease; and
- a second enzymatic component, comprising or consisting of a cellulolytic enzyme, preferably cellulase,
wherein the weight ratio of the weight of the first enzymatic component, preferably prolyl-specific protease, preferably prolyl-specific endoprotease, to the weight of the second enzymatic component, preferably cellulase, more preferably beta-glucanase, is:
- equal to or more than 0.1:1, more preferably equal to or more than 0.5:1, even more preferably equal to or more than 1:1, still more preferably equal to or more than 2:1, yet more preferably equal to or more than 3:1; and/or
- equal to or less than 1000:1, more preferably equal to or less than 100:1, even more preferably equal to or less than 50:1, still more preferably equal to or less than 20:1.
In a special preferred embodiment, the weight ratio of the weight of the first enzymatic component, preferably prolyl-specific protease, preferably prolyl-specific endoprotease, to the weight of the second enzymatic component, preferably cellulase, more preferably beta-glucanase, is about 4:1.

Preferably the cellulolytic enzyme, preferably cellulase, is present in an amount of equal to or more than 0.001% w/w, more preferably equal to or more than 0.005%w/w and still more preferably equal to or more than 0.01% w/w, and most preferably equal to or more than 0.05 % w/w, based on the total weight of the prolyl-specific protease, preferably a prolyl-specific endoprotease.

Preferably the cellulolytic enzyme, preferably cellulase, is present in an amount of equal to or less than 100% w/w, more preferably equal to or less than 50%w/w and still more preferably equal to or less than 10%w/w, and most preferably equal to or less than 5%w/w, based on the total weight of the prolyl-specific protease, preferably a prolyl-specific endoprotease. These ratios are advantageous in view of the differences in presence of polysaccharides and other components in the haze.

The composition according to the invention is preferably a composition, wherein the first enzymatic component and the second enzymatic component are derived from or produced by the same micro-organism, preferably the same bacterium or fungus, most preferably the same Trichoderma reesei.

Preferably the enzyme composition is a liquid composition. Preferably the enzyme composition further comprises a solvent.

Preferably the solvent comprises or consists of water. In addition to the water, one or more co-solvents may or may not be present. Preferably the composition comprises water and optionally one or more co-solvents. If a co-solvent is present, the solvent can comprise water and one or more co-solvents. Preferred co-solvents include tripropylene glycol methyl ether, dipropylene glycol methyl ether, propylene glycol methyl ether, diethylene glycol butyl ether, dipropylene glycol, Methylene glycol, 1 ,2- propanediol, N-ethyl-2-pyrroldinone, isopropanol, ethanol, ethyl lactate, 1 ,3-propanediol, and/or any combinations thereof.

The solvent may or may not comprise glycerol. Preferably the solvent comprises less than 50% v/v, more preferably less than 40% v/v, still more preferably less than 30 % v/v, even more preferably less than 20 % v/v, and most preferably less than 10 % v/v glycerol, based on the total volume of the solvent. Most preferably the solvent does not comprise glycerol. More recently glycerol has been subject to a lot of price volatility and therefore is less preferred. The use of the solution stabilizer and/or osmolyte as described herein advantageously allows one to reduce the amount of glycerol in the solvent whilst still obtaining good stability and/or activity and/or avoiding precipitation.

Preferably the composition has a pH value in the range from equal to or more than pH 1.0, more preferably from equal to or more than pH 1.2 to equal to or less than pH 6.0, more preferably to equal to or less than pH 5.5, still more preferably to equal to or less than pH 5.0, even more preferably to equal to or less than pH 4.5 and most preferably to equal to or less than pH 4.0.

More preferably the composition is a liquid composition having a pH in the range from equal to or more than pH 1.0, more preferably from equal to or more than pH 1.2 to equal to or less than pH 6.0, more preferably to equal to or less than pH 5.0, still more preferably to equal to or lower than pH 4.0, even more preferably to equal to or lower than pH 3.0 and most preferably to equal to or less than pH 2.5. More preferably the composition is a liquid composition having a pH in the range from pH 1.0 to pH 5.0, even more preferably in the range from pH 1.2 to pH 4.5.

In an alternative embodiment the enzyme composition can be provided as a frozen or freeze-dried form and be reconstituted before application by the addition of a solvent as described above during pre-processing.

In addition the enzyme composition may comprise one or more additional components, preferably chosen from the group consisting of:
- a salt, preferably chosen from the group consisting of sodium chloride, potassium chloride and ammonium sulphate;
- a carboxylic acid, preferably having in the range from equal to or more than 1 to equal to or less than 10 carbon atoms, preferably in the range from equal to or more than 1 to equal to or less than 7 carbon atoms, and/or any ester and/or any salt thereof, more preferably formic acid, acetic acid, diacetic acid, ascorbic acid, lactic acid, citric acid, propionic acid, oxalic acid, malic acid and/or fumaric acid, and/or any ester thereof and/or any salt thereof;
- a glyceryl monoacetate, glyceryl diacetate, glyceryl triacetate, glyceryl monopropionate, glyceryl dipropionate, glyceryl tripropionate, glyceryl monobutanoate, glyceryl dibutanoate, glyceryl tributanoate, glyceryl monolactate, glyceryl dilactate, glyceryl trilactate;
- a monosaccharide, a disaccharide and/or an alcohol-derivative or chloride-derivative of such a monosaccharide or disaccharide;
- a sorbitol, mannitol, inositol, trehalose, sucrose, mannose and/or sucrolose;
- a maltodextrin, xylan, mannan, fucoidan, galactomannan, chitosan, raffinose, stachyose, pectin, inulin, levan, graminan, amylopectin, and mixtures thereof.

As indicated above, the composition may or may not comprise glycerol.

In addition, the composition may or may not comprise benzoic acid or a derivative thereof.

The composition further may or may not comprise one or more additional enzymes in addition to the endoprotease enzyme, such as for example acetolactate decarboxylate (ALDC), aminopeptidase or trehalase, glucoamylase, xylanase, maltogenic alpha-amylase, pullulanase, catalase or transglucosidase. In a preferred embodiment the composition comprises one or more additional enzymes, preferably acetolactate decarboxylate (ALDC), glucoamylase and/or xylanase. In one more preferred embodiment the composition comprises or consists of the first enzymatic component, the second enzymatic component and xylanase. In a second more preferred embodiment the composition comprises or consists of the first enzymatic component, the second enzymatic component and acetolactate decarboxylate (ALDC). In a third more preferred embodiment the composition comprises or consists of the first enzymatic component, the second enzymatic component and glucoamylase.

### Other embodiments

The invention further provides a bacterial or fungal strain, preferably a *Trichoderma reesei* strain, functionally expressing:
- a first nucleic acid sequence encoding a first enzymatic component, comprising or consisting of a prolyl-specific protease, preferably a prolyl-specific endoprotease; and
- a second nucleic acid sequence encoding a second enzymatic component, comprising or consisting of a cellulolytic enzyme, preferably cellulase.

In such bacterial or fungal strain, preferably a *Trichoderma reesei* strain, preferably the first nucleic acid sequence is:
- operationally linked to a promoter; or
- present in 2, 3, 4, 5, 6 or more copies.

Hence, the invention also provides a bacterial or fungal strain, preferably *Trichoderma reesei* strain, functionally expressing:
- a first nucleic acid sequence encoding a first enzymatic component, comprising or consisting of a proplyl-specific protease, preferably a prolyl-specific endoprotease; and
- a second nucleic acid sequence encoding a second enzymatic component, comprising or consisting of a cellulolytic enzyme, preferably cellulase.
wherein the first nucleic acid sequence is:
- operationally linked to a promoter; or
- present in 2, 3, 4, 5, 6 or more copies.

The invention further provides a bacterial or fungal strain, preferably a *Trichoderma reesei* strain, functionally expressing:
- a first nucleic acid sequence encoding a recombinant first enzymatic component, comprising or consisting of a prolyl-specific protease, preferably a prolyl-specific endoprotease; and
- a second nucleic acid sequence encoding a natural occurring second enzymatic component, comprising or consisting of a cellulolytic enzyme, preferably cellulase.
In such bacterial or fungal strain, preferably a *Trichoderma reesei* strain, preferably the first nucleic acid sequence is:
- operationally linked to a promoter; or
- present in 2, 3, 4, 5, 6 or more copies.

The above may advantageously cause the bacterial or fungal strain, preferably a *Trichoderma* reesei strain, to produce the first enzymatic component in a higher amount than the second enzymatic component.

Preferably the first nucleic acid sequence is a heterologous nucleic acid sequence and the first enzymatic component is heterologous expressed. Preferably the second nucleic acid sequence is a endogenous nucleic acid sequence and the second enzymatic component is endogenous expressed.

### Process

The invention also provides a process for the preparation of an alcoholic beverage, preferably a beer, comprising the use of the above novel enzyme composition.

The invention further provides a process for the production of an alcoholic beverage for human consumption, comprising the addition of a composition according to the invention or the addition of a bacterial strain according to the invention. Preferably such a process is a simultaneous saccharification and fermentation process ("SSF")

More preferably a process is provided for the production of an alcoholic beverage for human consumption, comprising the addition of a composition as described above or the addition of a bacterial or fungal strain, preferably a *Trichoderma reesei* strain, as described above.

A large variety of beer brewing processes in used in the world. However, every beer brewing process typically comprises saccharification and fermentation. During the saccharification a feedstock, such as starch, can be converted into saccharides. During fermentation such saccharides can be converted into ethanol (alcohol). Depending on the efficiency of the saccharification, it may be beneficial to have the second enzymatic component, preferably cellulase, present during the fermentation, in line with the invention. The invention therefore also provides a beer brewing process comprising the addition of a composition according to the invention or the addition of a bacterial strain according to the invention, preferably during the saccharification or fermentation step.

Most preferably, the process is a beer brewing process, wherein a composition according to the invention is added before or during fermentation, more preferably before fermentation, most preferably to the wort.

In an especially preferred embodiment such a beer brewing process is a simultaneous saccharification and fermentation process ("SSF"). Most preferably the composition is added in such a process during the simultaneous saccharification and fermentation.

The composition according to the invention is thus preferably a composition that can be added to a process for the production of an alcoholic beverage for human consumption, more preferably a composition that can be added to a beer brewing process, which composition comprises or consists of:
- a first enzymatic component, comprising or consisting of glutamine-specific protease and/or prolyl-specific protease, preferably a glutamine-specific endoprotease and/or prolyl-specific endoprotease; and
- a second enzymatic component, comprising or consisting of a cellulolytic enzyme, preferably cellulase.

Preferably the first enzymatic component is added to the processes as described herein in a dosage of equal to or more than 0.05 gram per hectoliter (gr/hl), more preferably equal to or more than 0.1 gr/hl, still more preferably equal to or more than 0.5 gr/hl and most preferably equal to or more than 1.0 gr/hl and/or preferably equal to or less than 100gr/hl, more preferably equal to or less than 50 gr/hl, most preferably equal to or less than 10 gr/hl. Preferably the first enzymatic component is added before or during fermentation, preferably before fermentation, preferably to the wort in a beer brewing process.

Preferably the second enzymatic component is added to the processes as described herein in a dosage of equal to or more than 0.001 gram per hectoliter (gr/hl), more preferably equal to or more than 0.01 gr/hl, still more preferably equal to or more than 0.05 gr/hl and most preferably equal to or more than 0.1 gr/hl and/or preferably equal to or less than 50gr/hl, more preferably equal to or less than 10 gr/hl, even more preferably equal to or less than 5 gr/hl and most preferably equal to or less than 1 gr/hl.

Most preferably the first enzymatic component and the second enzymatic component are added in a weight ratio, wherein the weight ratio of the weight of the first enzymatic component, preferably prolyl-specific protease, preferably prolyl-specific endoprotease, to the weight of the second enzymatic component, preferably cellulase, more preferably beta-glucanase, is:
- equal to or more than 0.1:1, more preferably equal to or more than 0.5:1, even more preferably equal to or more than 1:1, still more preferably equal to or more than 2:1, yet more preferably equal to or more than 3:1; and/or
- equal to or less than 1000:1, more preferably equal to or less than 100:1, even more preferably equal to or less than 50:1, still more preferably equal to or less than 20:1.
The addition of the first enzymatic component and the second enzymatic component in such a weight ratio is especially advantageous in a process for the production of an alcoholic beverage for human consumption, more preferably a beer brewing process. Most preferably the first enzymatic component and the second enzymatic component are added in such a ratio before or during fermentation, for example to a wort.

The addition of said enzyme composition in a process for the production of an alcoholic beverage for human consumption, especially a beer brewing process, can positively affect the taste of the produced beverage, such as a beer, by increasing the amount of mono-, di-, and tri-saccharides which have a sweet taste and enhance fruity flavours. It is therefore preferable that the addition of the enzyme composition in a process for the production of an alcoholic beverage for human consumption, especially a beer brewing process, increases the amount of mono-, di-, and/or tri-saccharides in the produced alcoholic beverage, such as a beer.

The addition of the enzymatic composition, in particular a composition comprising both a prolyl-specific endoprotease and a cellulase from *Trichoderma reesei,* in a process for the production of an alcoholic beverage for human consumption, especially a beer brewing process, can further improve the removal of fine particulate matter as is shown in the experimental section. The addition of a composition combining prolyl endoprotease and cellulase during beer fermentation can reduce the fouling of membranes during beer filtration and the filtration can advantageously be operated at an increased pressure and speed, gaining an economic advantage.

### Alcoholic beverage

The invention further provides an alcoholic beverage for human consumption obtained or obtainable by such a process.

More preferably the invention provides an alcoholic beverage comprising, preferably at least partly inactivated:
- glutamine-specific and/or proline-specific protease, preferably glutamine -specific and/or proline-specific endoprotease, preferably as described herein above; and
- cellulase, preferably as described herein above.

### Examples

### Materials and Methods

### Materials

In the examples, the following materials were used:
- Brewers Clarex is a product of DSM Food Specialties and contains a prolyl-specific endoprotease derived from a selected self-cloned strain of *Aspergillus niger.*
- Brewers Clarex XF is a product of DSM Food Specialties and contains a prolyl-specific endoprotease derived from a selected self-cloned strain of *Aspergillus niger.*
- Filtrase NL-Fast is a product of DSM Food Specialties and contains a fungal cellulase, more specifically an endo-1,3 (4)- β-glucanase (a beta-glucanase), derived from a selected strain of *Talaromyces emersonii.*
- Laminex BG3 is a product of IFF and contains a fungal cellulase (beta-glucanase) from *Trichoderma reesei.*
- Filtrase BR-X L is a product of DSM Food Specialties and contains a fungal cellulase, more specifically an endo-1,3 (4)- β-glucanase (a beta-glucanase), derived from a selected strain of Talaromyces emersonii and a hemicellulose, more specifically an endo-1,4-β-xylanase (a xylanase), derived from a selected strain of *Disporotrichum dimorphosporum.*
- Glucanex 100G is a product of Novozymes and contains a fungal cellulase, more specifically a beta-glucanase, from *Trichoderma harzianum.*
- Saflager S023: a bottom fermenting dried yeast *(Saccharomyces pastorianus)* from Lasaffre (Fermentis), which is suited for direct pitching.
- Heineken lager beer: a 5% ABV pale lager produced by Heineken
- Malt: EBC 21st EBC (European Brewery Convention) standard malt.
- Barley: IFBM (Institut Français des Boissons de la Brasserie et de la Malterie) 2 RS Barley-rgt planet crop 2020.

### Analytical methods

### Filtration

After maturation, the samples were centrifuged at 11,600g (Sorvall RC6 Plus, rotor F10S-6*500Y) and 4°C for 10 min. The supernatant was poured off and degassed by magnetic stirring at rT for 1 hour. The recovered beer was diluted five times on weight by the addition of similarly degassed Heineken lager beer. Prior to filtration, the diluted sample and the stainless-steel filter house of EDF-14-2 Seitz filtration system were cooled in ice-water.

The filter system was assembled according to the manufacturer's instructions by clamping the filter house to the bottom plate consisting of a perforated disc at the inner part and a connector (an 3/8-inch connector - ball valve - 3/8-inch connector and male luer lock (Z514691-1EA from sigma), stainless steel and Teflon taped) affixed to a tripod at the outer part. A volume of 250 ml of cooled sample was poured into the pre-cooled filter house. The ball valve was opened to allow about 10 grams of sample to flow through by gravity after which the switch was closed ensuring complete fill of the connector exit without any air bubbles. The filter (Acrodisc PES 0.45µm 25mm, PN 4508; Pall Corporation) was rinsed by backflush with 5 ± 1 ml degassed Heineken lager beer by attaching it to a disposable plastic syringe. The filter was slowly rinsed with the remainder of the beer during unscrew of the filter from the syringe to prevent air (bubbles) in the filter and the connection. The filter was attached to male luer lock at the filtration unit exit. The top plate of the filtration system was clamped to the filter house following the manufacturer's instructions. The vent valve at the top plate was closed, and the filtration system was connected to a pressure valve after which a pressure of 1 bar was applied. A balance with an accuracy of 0.1g was put below the filtration system. A beaker was put on the balance. The automatic weight recording was started from weight 0. Thereafter, the valve is opened at an exact timepoint of the automated logging, such that the weight is recorded immediately. Every 10 seconds the weight is recorded for 6-7 minutes in total.

The filtrate weight, transferred in arbitrary units (U), in time was used to determine the fouling. The fouling of the 0.45 µm filter, expressed in hour⁻¹ * bar⁻¹, is the inverse of the obtained slope of the applied linear relationship (applied complete blocking as filtration model) between flux, expressed in M(illion)U/(m²*hr*bar), against the filter load in MU/m².

### Determination of Specific Gravity, Plato, ABV and ADF

The specific gravity (SG) and alcohol content (Alcohol By Volume) of wort and fermentation samples after maturation was determined using a digital density meter (Anton Paar; type DMA58) using supplier instructions. The specific gravity was measured at 20 °C.

The Apparent Degree of Fermentation (ADF) was calculated according to ADF = (Original extract - Apparent extract) / Original Extract *100

The original extract expressed in degrees Plato (°P) was calculated by the formula: Extract = - 460,234 + 662,649 x (SG) - 202,414 x (SG)2 wherein SG is the specific gravity of the wort (EBC method 8.3 Extract of wort -2004).

The apparent extract expressed in degrees Plato (°P) was calculated by the formula: Extract = -460,234 + 662,649 x (SG) - 202,414 x (SG)2 wherein SG is the specific gravity of sample after fermentation and maturation (EBC method 9.4 Original, real and apparent extract and original gravity of beer - 2004).

### Analysis of fermentable sugars

The analysis for fermentation samples after maturation was performed on a High Performance Anion Exchange Chromatography system from Thermo Fisher Scientific (Dionex), coupled with pulsed amperometric detection (HPAEC-PAD) on a CarboPac PA20 column, with Amino Trap guard column and Borate Trap pre-column. The mobile phase was a gradient consisting of 3 solutions. The gradient settings to analyze the fermentable sugars with this system are depicted below in Table 1.

**Table 1_{:} Gradient settings for sugar analysis:**

| **Time [min]** | **% A** | **% C** | **% D** | **Curve** |
|---|---|---|---|---|
| - 15 | 80 | 20 | 0 | 5 |
| 0 | 80 | 20 | 0 | 5 |
| 30 | 64 | 16 | 20 | 5 |
| 31 | 0 | 0 | 100 | 5 |
| 36 | 0 | 0 | 100 | 5 |
| 37 | 80 | 20 | 0 | 5 |

| | | | | |
|---|---|---|---|---|
| Mobile phase A: Milli Q purified water Mobile phase C: 500 mM NaOH Mobile phase D: 100 mM NaOH/ 1 M NaOAc | | | | |

The peak area of the fermentable (glucose, maltose and maltotriose) sugars present in the samples were quantified using calibration curves of the references glucose, maltose and maltotriose.

### Analysis of beer turbidity (haze)

Haze was measured on fermentation samples after maturation with a Haffman's VOS Rota 90/25 dual angle turbidity meter. This equipment measures the scattered light caused by particles. Particles smaller than 1 µm mainly cause scattered light and are measured under a 90° angle. Particles larger than 1 µm mainly cause forward-scattered light and are measured under a 25° angle. The measurement was performed in the provided cuvette by the manufacturer of the used equipment (EBC method 9.29 Haze in beer: calibration of haze meters-2015).

### Analysis of glucan

The product obtained after the reaction with the cellulases (glucanases) is a glucan. The level of glucan in wort and fermentation samples after maturation was determined by Enzytec Color GlucaTest from R-Biopharm AG (EBC 4.16.3, MEBAK 3.1.4.9.2) according to the manufacturer's instructions (protocol 06.04.2017). This method is accepted and recommended for inclusion in Analytical-EBC since 2005.

### Example 1: Use of the combination of a prolyl endoprotease and a cellulase during beer fermentation

For this example mashing was performed with 80 grams of grist consisting of 60w% disk milled malt (EBC 21^{st} EBC standard malt) and 40w% hammer milled barley (IFBM 2 RS Barley-rgt planet crop 2020) to which 200 grams of tap water was added and mixed.

The suspension was brought into a conversion vessel prewarmed at 48°C in LB Electronic from Lochner. A volume of 1 ml calcium chloride (liquid stock of 0.61 M) was added, and the pH was corrected to pH 5.4 using 80% lactic acid whiling mixing at 100 rpm. Thereafter the mashing profile was applied in LB Electronic as depicted in Table 2. In the meantime, completion of the saccharification was checked by performing an iodine test (Lugal solution; 32922 from Sigma, according to Lugol). At the end of mashing, no starch was detected by the iodine test.

**Table 2: Applied mashing profile for grist consisting of 60w% malt and 40w% barley**

| Mash profile for mashing | °C | min |
|---|---|---|
| 1. rest | 48 | 35 |
| 2. rest | 65 | 50 |
| 3. rest | 72 | 30 |
| 4. rest | 78 | 5 |
| Ramp [°C per min] | 1.0 | |
| Total rest time [min] | 120 | |
| Total heating time [min] | 30 | |
| Total [min] | 150 | |

After completion of the mash scheme, the weight of the mash suspension was adjusted to 450 grams with hot tap water. The suspension was stirred and poured onto a folded filter (Macherey-Nagel; MN 614 ¼ Ø 320 mm REF 527032) in a glass funnel placed on a 500 ml Scott flask for collection of the filtrate. The filtration was stopped when the filter bed in the folded filter ran dry by gravity. The first 100 ml filtrate was collected and was fed back on top of the filter bed. The total collected filtrate, wort, was cooled down to room temperature.

The resulting wort solution had a gravity of 14.6 °P, a specific gravity of 1.059582 g/cm3 and a fermentable sugar content of 95.8 g/L.

The total wort generated via 16 individual mashes was divided into 300-gram portions in Scott flasks. The wort was cooled to 14°C after which the yeast (Saflager S23, Fermentis) was dosed at 100 g/hl and the enzyme(s) was (were) applied according to Table 3. Thereafter, the fermentation was performed for 8 days at 14°C and 100 rpm using an ANKOM gas fermentation system During fermentation, the cumulative pressure and the temperature were logged using the ANKOM RF Gas Production System (Ankom Technology).

**Table 3: Dosage of commercial enzyme to cold wort**

| Experiment number | Dosage of commercial enzyme (g/hl) | | | | | |
|---|---|---|---|---|---|---|
| | Filtrase NL Fast | Laminex BG3 | Filtrase BRXL | Glucanex 100G | Brewers Clarex | Brewers Clarex XF |
| 1A | 0.5 | - | - | - | 2 | - |
| 1B | - | 0.5 | - | - | 2 | - |
| 1C | - | - | 0.5 | - | 2 | - |
| 1D | - | - | - | 0.5 | 2 | - |
| Control 1 | - | - | - | - | 2 | - |
| 2A | 0.5 | - | - | - | - | 2 |
| 2B | - | 0.5 | - | - | - | 2 |
| 2C | - | - | 0.5 | - | - | 2 |
| 2D | - | - | - | 0.5 | - | 2 |
| Control 2 | - | - | - | - | - | 2 |
| Control 0 | - | - | - | - | - | - |

At the end of the fermentation, beer maturation was performed by refrigeration at 0°C for 3 days. After maturation, the samples were centrifuged at 11,600g and 4°C for 10 min to remove the yeast. The supernatant was poured off and degassed by magnetic stirring at rT for 1 hour. This clarified beer was used for the compositional analysis and the physical analysis.

### Results

### Improvement of taste

To illustrate the improvement in taste, a compositional analysis was carried out.

The Apparent Degree of Fermentation (ADF) of all samples varied between 78 and 82%, and alcohol content between 6.20 and 6.45% The presence of fermentable sugars in the final beer is depicted in Table 4.

**Table 4: Presence of glucose, maltose and maltotriose in the samples. Total fermentable sugars is the sum of these.**

| | Glucose | Maltose | Malto-triose | Fermentable sugars |
|---|---|---|---|---|
| | [g/l] | [g/l] | [g/l] | [g/l] |
| Experiment 1A | 0.16 | 4.43 | 1.84 | 6.42 |
| Experiment 1B | 0.24 | 6.18 | 2.51 | 8.92 |
| Experiment 1C | 0.23 | 5.98 | 2.32 | 8.53 |
| Experiment 1D | 0.16 | 4.70 | 1.95 | 6.81 |
| Control 1 | 0.15 | 4.32 | 1.85 | 6.32 |
| Experiment 2A | 0.17 | 4.87 | 2.00 | 7.04 |
| Experiment 2B | 0.22 | 5.29 | 2.09 | 7.60 |
| Experiment 2C | 0.20 | 4.00 | 1.59 | 5.79 |
| Experiment 2D | 0.15 | 3.96 | 1.58 | 5.69 |
| Control 2 | 0.14 | 3.75 | 1.62 | 5.51 |
| Control 0 | 0.15 | 4.27 | 1.68 | 6.10 |

The sugar composition of the final beer can have an influence on the taste perception. Mono-, di-, and tri-saccharides have a sweet taste, and are known to enhance fruity flavours. Hence, it is expected that an increase in the presence of fermentable sugars in the final beer, as can be detected in Experiments 1B and 2B that are treated with a prolyl endoprotease and a cellulase, will favourably influence the flavour of the beer.

In addition, glucan could be clearly detected in each of the control samples, "0", "1" and "2", in amounts ranging from 20 to 35 mg/L. Glucans can be problematic for filtration and may give haze/precipitates in the final beer.

However, the glucan was below detection limit in all experimental samples that contained cellulase activity. This indicates that all cellulases had been active during beer fermentation to further break down the glucan into the glucose. Glucose is sweeter than glucan and may also enhance fruity flavours, as explained also above. It can thus be concluded that during the fermentation in experiments 1A, 1B, 1C, 1D and 2A, 2B, 2C and 2D, in addition to the prolyl endoprotease activity, there has also been cellulase activity. The combination of these led to an improved break-down of glucan into the desired glucose, and an improved taste.

### Improvement in the removal of fine particulate matter

To illustrate the improvement in removal of fine particulate matter, a physical analysis was carried out.

For testing the effect of the enzyme treatment on the possible presence of fine particulate matter in the fermentation and monitoring the speed of the removal of such matter, haze at the end of fermentation after maturation and filterability of the clarified beer were tested.

### a) Testing for the presence of fine particulate matter

Haze measured at the end of fermentation after maturation is presented in Table 5

**Table 5: Haze formation measured at a 90° or 25° angle.**

| | **H90** | **H25** |
|---|---|---|
| Experiment 1A | 4.69 | 5.48 |
| Experiment 1B | 8.18 | 13.34 |
| Experiment 1C | 6.15 | 9.97 |
| Experiment 1D | 4.30 | 5.31 |
| Control 1 | 5.29 | 6.81 |
| Experiment 2A | 3.97 | 5.42 |
| Experiment 2B | 5.46 | 7.94 |
| Experiment 2C | 3.25 | 4.27 |
| Experiment 2D | 3.16 | 2.86 |
| Control 2 | 4.52 | 4.86 |
| Control 0 | out of range | out of range |

This experiment shows that addition of a prolyl endoprotease has a significant effect on reduction of the haze in beer at the end of fermentation. In control 0, without added prolyl endoprotease, the haze formation was too high and was out of range for the measurement instrument. The haze in all samples with added prolyl endoprotease could however be measured within range. The combination of prolyl endoprotease with cellulases in some instances showed either a slight increase, or a slight further decrease in haze, dependent on the origin of the cellulase. However, any haze formation at the end of fermentation may not be significant for haze formation in the final bottled beer. An important step here is the removal of fine particulate matter in the filtration step is of importance.

That is, the above results show that at the end of fermentation after maturation fine particulate matter may be present and the removal of such is desired.

### b) Filtration

In a regular beer production, the fine particulate matter is removed by a filtration step after fermentation and maturation. To test the effect of the enzymatic treatment on the capacity and flux of the beer filtration the samples were first diluted 5x with commercial degassed Heineken lager. This dilution allows a more accurate assessment since the time until blocking of the filters is increased. Prior to filtration, the diluted sample and the stainless-steel filter house of EDF-14-2 Seitz filtration system were cooled in ice-water.

Amount of filtrate was followed in time and the results are presented in Figure 1. All samples that contain cellulase in combination with prolyl endoprotease show an improvement in the filtrate yield before blockage of the filter. The speed of filtration, compared to the 3 control samples that had not been treated with cellulase, was also improved.

Fouling rate was calculated from the data and is depicted in Table 6. Clearly with all samples where prolyl endoprotease was combined with cellulase during beer fermentation, the fouling of membranes during clear beer filtration was reduced. Hence, filtration can be operated at an increased speed (cq pressure) gaining a process advantage compared to a regular clear beer filtration. Surprisingly, there is a clear difference in the extent to which different cellulases reduce filter fouling. Most effective in reducing fouling seems to be the cellulase of Trichoderma reesei. This is surprising since in the samples treated with the cellulase of Trichoderma reesei the haze formation before filtration was higher than in the controls with only prolyl endoprotease (see Table 5).

**Table 6: Fouling rate of filters used to clarify the different beer samples.**

| | Fouling (hour*bar)-1 |
|---|---|
| Experiment 1A | 96.5 |
| Experiment 1B | 36.6 |
| Experiment 1C | 100.3 |
| Experiment 1D | 139.9 |
| Control 1 | 179.4 |
| Experiment 2A | 91.1 |
| Experiment 2B | 37.4 |
| Experiment 2C | 81.9 |
| Experiment 2D | 137.5 |
| Control 2 | 187.4 |
| Control 0 | 215.5 |

## Claims

1. A composition, for addition to a process for the production of an alcoholic beverage for human consumption, comprising or consisting of:
- a first enzymatic component, comprising or consisting of a glutamine-specific protease and/or prolyl-specific protease, preferably a glutamine-specific endoprotease and/or prolyl-specific endoprotease, most preferably a prolyl-specific endoprotease; and
- a second enzymatic component, comprising or consisting of a cellulolytic enzyme, preferably cellulase,
wherein the second enzymatic component is derived from and/or produced by a *Trichoderma reesei.*

2. The composition according to claim 1, wherein the first enzymatic component is derived from and/or produced by a micro-organism, preferably a bacterium or fungus, most preferably a *Trichoderma reesei.*

3. The composition according to claim 1 or 2,
wherein the first enzymatic component comprises or consists of a prolyl-specific endoprotease and wherein the cellulolytic enzyme, preferably cellulase, is present in an amount of equal to or less than 50%w/w, based on the total weight of the prolyl-specific endoprotease.

4. The composition according to any of the preceding claims, wherein
- the first enzymatic component consists of a prolyl-specific endoprotease; and
- the second enzymatic component consists of a cellulolytic enzyme, preferably cellulase,
wherein the second enzymatic component is derived from and/or produced by a *Trichoderma reesei.* wherein the composition not comprises one or more additional enzymes.

5. The composition according to any one of the preceding claims, wherein the composition is for addition during the saccharification and/or fermentation step of a beer brewing process.

6. The composition according to any one of the preceding claims, wherein the composition is for improving the removal of fine particulate matter in a fermentation broth and/or fermentation tank and/or for improving the taste of the alcoholic beverage product

7. The composition according to any one of the preceding claims, wherein the first enzymatic component and the second enzymatic component are derived from or produced by the same micro-organism, preferably the same bacterium or fungus, most preferably the same Trichoderma reesei.

8. The composition according to any one of the preceding claims, wherein the first enzymatic component has its optimum activity at a pH in the range from equal to or more than pH 1.0, more preferably from equal to or more than pH 1.2 to equal to or less than pH 6.0, more preferably to equal to or less than pH 5.5, still more preferably to equal to or less than pH 5.0, even more preferably to equal to or less than pH 4.5 and most preferably to equal to or less than pH 4.0.

9. The composition according to any one of the preceding claims, wherein the second enzymatic component has its optimum activity at a pH in the range from equal to or more than pH 1.0, more preferably from equal to or more than pH 1.2 to equal to or less than pH 6.0, more preferably to equal to or less than pH 5.5, still more preferably to equal to or less than pH 5.0, even more preferably to equal to or less than pH 4.5 and most preferably to equal to or less than pH 4.0.

10. The composition according to any one of the preceding claims, wherein the composition comprises water and optionally one or more co-solvents.

11. The composition according to any one of the preceding claims, wherein the composition has a pH value in the range from equal to or more than pH 1.0, more preferably from equal to or more than pH 1.2 to equal to or less than pH 6.0, more preferably to equal to or less than pH 5.5, still more preferably to equal to or less than pH 5.0, even more preferably to equal to or less than pH 4.5 and most preferably to equal to or less than pH 4.0.

12. The composition according to any one of the preceding claims, wherein the composition comprises one or more additional components preferably chosen from the group consisting of:
- a salt, preferably chosen from the group consisting of sodium chloride, potassium chloride and ammonium sulphate;
- a carboxylic acid, preferably having in the range from equal to or more than 1 to equal to or less than 10 carbon atoms, preferably in the range from equal to or more than 1 to equal to or less than 7 carbon atoms, and/or any ester and/or any salt thereof, more preferably formic acid, acetic acid, diacetic acid, ascorbic acid, lactic acid, citric acid, propionic acid, oxalic acid, malic acid and/or fumaric acid, and/or any ester thereof and/or any salt thereof;
- a glyceryl monoacetate, glyceryl diacetate, glyceryl triacetate, glyceryl monopropionate, glyceryl dipropionate, glyceryl tripropionate, glyceryl monobutanoate, glyceryl dibutanoate, glyceryl tributanoate, glyceryl monolactate, glyceryl dilactate, glyceryl trilactate;
- a monosaccharide, a disaccharide and/or an alcohol-derivative or chloride-derivative of such a monosaccharide or disaccharide;
- a sorbitol, mannitol, inositol, trehalose, sucrose, mannose and/or sucrolose;
- a maltodextrin, xylan, mannan, fucoidan, galactomannan, chitosan, raffinose, stachyose, pectin, inulin, levan, graminan, amylopectin, and mixtures thereof;

13. The composition according to any one of the preceding claims, wherein the composition does or does not comprise glycerol.

14. The composition according to any one of the preceding claims, wherein the composition does or does not comprise benzoic acid or a derivative thereof.

15. A bacterial or fungal strain, preferably a *Trichoderma reesei* strain, functionally expressing:
- a first nucleic acid sequence encoding a first enzymatic component, comprising or consisting of a prolyl-specific endoprotease; and
- a second nucleic acid sequence encoding a second enzymatic component, comprising or consisting of a cellulolytic enzyme, preferably cellulase, wherein the second enzymatic component is derived from *Trichoderma reesei.*

16. The bacterial or fungal strain, preferably *Trichoderma reesei* strain, according to claim 15, functionally expressing:
- a first nucleic acid sequence encoding a first enzymatic component, comprising or consisting of glutamine-specific protease and/or prolyl-specific protease, preferably a glutamine-specific endoprotease and/or prolyl-specific endoprotease; and
- a second nucleic acid sequence encoding a second enzymatic component, comprising or consisting of a cellulolytic enzyme, preferably cellulase.
wherein the first nucleic acid sequence is:
- operationally linked to a promoter; or
- present in 2, 3, 4, 5, 6 or more copies.

17. The bacterial or fungal strain, preferably *Trichoderma reesei* strain, according to claim 15 or 16, wherein the first enzymatic component is produced in a higher amount than the second enzymatic component.
